# EUROPÄISCHE PATENTSCHRIFT

(11) **EP 0 566 892 B1**
(45) Veröffentlichungstag und Bekanntmachung des Hinweises auf die Patenterteilung: **01.09.1999**
(21) Anmeldenummer: 93104931.6
(22) Anmeldetag: 25.03.1993
(51) Int. Cl.: A61L 2/20

(54) **Sterilisiergasgemisch**
Sterilisant gas mixture
Mélange de gaz stérilisants

(30) Priorität: 21.04.1992 DE 4213091
(43) Veröffentlichungstag der Anmeldung: 27.10.1993
(73) Patentinhaber: SOLVAY (Société Anonyme), 1050 Bruxelles (BE)
(72) Erfinder: Deger, Hans-Matthias, Dr., W-6238 Hofheim-Wallau (DE); Henrici, Rainer, W-6392 Neu-Anspach (DE); Preisegger, Ewald, W-6085 Nauheim (DE)
(74) Vertreter: Jacques, Philippe

(56) Entgegenhaltungen:
- EP-A- 0 478 115
- US-A- 4 971 716
- DATABASE WPIL Section Ch, Week 9122, Derwent Publications Ltd., London, GB; Class E16, AN 91-159429 & JP-A-3 093 890 (DAIKIN KOGYO)

## Beschreibung

Die vorliegende Erfindung betrifft ein Sterilisiergasgemisch für die Kaltgassterilisation.

Unter Sterilisation soll die Abtötung bzw. Inaktivierung von Insekten, Bakterien, Viren, Pilzen und anderen Mikroorganismen verstanden werden. Die abgetöteten bzw. inaktivierten Organismen werden bei dieser Sterilisierung nicht abgetrennt.

Die in der Technik gebräuchliche Sterilisation durch kochendes Wasser, heißen Wasserdampf oder heiße Luft ist nicht generell einsetzbar. In bestimmten Bereichen der Medizin müssen Geräte, Ausrüstungen und Werkzeuge sterilisiert werden, die entweder empfindlich gegen Feuchtigkeit sind, oder den Temperaturen von 100 - 180 °C einer Heißgassterilisation nicht standhalten.

In diesen Fällen wird eine Kaltgassterilisation durchgeführt, bei welcher man Ethylenoxid und Propylenoxid als Sterilisiergas verwendet. Diese Alkenoxide sind hocheffiziente Sterilisiermedien, die bereits bei niedrigen Temperaturen volle Wirkung entfalten. Die zu sterilisierenden Gegenstände werden bei der Kaltgassterilisation thermisch nicht beansprucht und die Alkenoxide sind nach Beendigung des Sterilisationsvorganges wegen ihrer hohen Flüchtigkeit vom sterilisierten Artikel schnell entfernbar. Obwohl Ethylenoxid und Propylenoxid allein für die Sterilisation verwendet werden könnten, werden diese aufgrund der Gefahr der Bildung zündfähiger Gemische mit Luft meist in Mischung mit einem phlegmatisierenden Gas eingesetzt. Als phlegmatisierendes Gas für Sterilisiergasgemische auf der Basis von Ethylenoxid ist Kohlendioxid bekannt. Zur Vermeidung der Bildung zündfähiger Gemische mit Luft können bei der Phlegmatisierung mit CO₂ lediglich bis zu höchstens 7 mol% Ethylenoxid als Sterilisiergasgemisch eingesetzt werden.

Deshalb muß die Sterilisation entweder bei höheren Drucken oder über längere Kontaktzeiten hinweg durchgeführt werden. Außerdem kann, bedingt durch den großen Dampfdruckunterschied von CO₂ und Ethylenoxid, das aus der Flüssigphase eines Vorratslagerbehälters entnommene Sterilisiergasgemisch bei zunehmender Dampfphase und abnehmender Flüssigphase im Vorratsbehälter mit Ethylenoxid angereichert sein, wodurch die Gefahr einer Bildung zündfähiger Gemische mit Luft besteht.

Es wurde deshalb vorgeschlagen, Dichlordifluormethan (R 12) als phlegmatisierendes Gas dem Sterilisiergasgemisch auf Basis von Ethylenoxid zuzumischen. Weiterhin wurde vorgeschlagen (US-A-49716716), Octafluorcyclobutan als phlegmatisierendes Gas zuzumischen. In EP-A-478115 wurde Pentafluorethan als phlegmatisierendes Gas vorgeschlagen.

Aufgrund der Fluorchlorkohlenwasserstoff-Ozon-Problematik darf Dichlordifluormethan (R 12) für diesen Zweck nicht mehr eingesetzt werden.

Es war daher die Aufgabe gestellt, ein Sterilisiergasgemisch für die Kaltgassterilisation zur Verfügung zu stellen, welches kein Dichlordifluormethan (R 12) enthält, kein zündfähiges Gemisch mit Luft bildet und das Sterilisieren in kurzer Zeit ohne die Anwendung von Überdruck oder erhöhter Temperaturen erlaubt.

Diese Aufgabe wird gelöst durch das erfindungsgemäße Sterilisiergasgemisch für die Kaltgassterilisation, welches kein zündfähiges Gemisch mit Luft bildet, bestehent aus Alkenoxid und 1,1,1,2,3,3,3-Heptafluorpropan (R 227).

Das erfindungsgemäße Sterilisiergasgemisch besteht wahlweise aus
a) 12 bis 22 mol% Ethylenoxid und 78 bis 88 mol% 1,1,1,2,3,3,3-Heptafluorpropan (R 227)
   oder
b) 12 bis 22 mol% Propylenoxid und 78 bis 88 mol% 1,1,1,2,3,3,3-Heptafluorpropan (R 227).
   Das erfindungsgemäße Sterilisiergasgemisch kann wahlweise auch noch dadurch abgeändert sein, daß
c) das Alkenoxid eine Mischung aus Ethylenoxid und Propylenoxid ist;
d) es Inertgase in Mengen bis zu 20 Volumen% enthält;
e) das Inertgas Stickstoff, Kohlendioxid oder ein Edelgas ist.

Ein weiterer Vorteil des erfindungsgemäßen Sterilisiergasgemisches mit 1,1,1,2,3,3,3-Heptafluorpropan (R 227) als Phlegmatisierungsmittel liegt darin, daß insbesondere bei Sterilisiergasgemischen auf der Basis von Ethylenoxid ein deutlich geringerer Siedetemperaturabstand vorliegt. Hierdurch wird die Gefahr einer Konzentrationsverschiebung in Richtung Ethylenoxid-Anreicherung bei zunehmendem Entleerungsgrad des Vorratsbehälters verringert. Ethylenoxid siedet bei 10,7 °C. Die Siedetemperatur von Dichlordifluormethan (R 12) liegt bei -29,8 °C, die von 1,1,1,2,3,3,3-Heptafluorpropan (R 227) bei -17,4 °C. Dies bedeutet, daß im erfindungsgemäßen Sterilisiergasgemisch geringere Konzentrationsverschiebungen als beim bisher eingesetzten Gemisch aus Ethylenoxid und R 12 eintreten werden.

Im Sterilisiergasgemisch auf Basis einer Ethylenoxid/Propylenoxid-Mischung mit R 227 ist die Siedetemperatur des R 227 (-17,4 °C) günstiger als die von R 12 (-29,8 °C), weil die Siedetemperatur des R 227 (-17,4 °C) sowohl den Siedepunkten des Ethylenoxids (10,7 °C) und des Propylenoxids (35 °C) als auch dem Siedepunktbereich der Ethylenoxid/Propylenoxid-Mischung näher liegt. Der Partialdruck vom Propylenoxid in der Ethylenoxid/Propylenoxid-Mischung liegt in einer Größenordnung, bei welcher die Ethylenoxid/Propylenoxid-Mischung ohne eine zusätzliche Erwärmung aus dem Vorratsbehälter entnommen werden kann, was bei der alleinigen Anwendung von Propylenoxid im Sterilisiergasgemisch nicht immer der Fall ist. Sollte der Gesamtdruck der erfindungsgemäßen Sterilisiergasmischung in flüssiger Phase bei vorgegebener Temperatur zu klein sein, so kann der Druck im Vorratsbehälter durch Inertgaszusatz erhöht werden, ohne daß eine gravierende Auswirkung auf die Bildung zündfähiger Gemische mit Luft eintritt.

Die erfindungsgemäße Sterilisiergasmischung kann zur Sterilisierung einer Vielzahl unterschiedlicher Artikel verwendet werden. Beispiele aus dem Bereich medizinischer Ausrüstungen und Materialien sind beispielsweise Endoskope, Kunststoffgegenstände wie Elastomerdichtungen an medizinischen Apparaten, Rohrmaterial, Inkubatoren, Herzschrittmacher, Gummierzeugnisse wie beispielsweise Schläuche, Handschuhe, Katheter, Instrumente wie Injektionsnadeln und Skalpelle und vieles andere mehr. Anwendungsbeispiele außerhalb des medizinischen Bereiches sind beispielsweise die Sterilisation von Fellen, Papiererzeugnissen, Transportbehältern oder Frachträumen von Flugzeugen, Zügen und Schiffen.

Das erfindungsgemäße Sterilisiergasgemisch ist wirksam gegen Insekten, Bakterien, Viren, Pilze und viele andere Mikroorganismen.

Das erfindungsgemäße Sterilisiergasgemisch kann durch bekannte Mischungstechniken hergestellt werden, die jedem auf diesem Gebiet tätigen Fachmann vertraut sind. Z.B. kann jede Komponente der Sterilisiergasmischung separat in einen Vorratsbehälter gepumpt werden, wobei die Einstellung des Mischungsverhältnisses über Wägung des Vorratsbehälters oder über die Messung des Volumenstromes jeder Einzelkomponente erfolgen kann. Die Vermischung der Einzelkomponenten kann im Vorratsbehälter durch Umpumpen vorgenommen werden, bis eine homogene Mischung erreicht ist. Die genaue Einstellung des Mischungsverhältnisses kann anschließend durch eine analytische Untersuchung kontrolliert werden.

Als Vorratsbehälter für das erfindungsgemäße Sterilisiergasgemisch können die bisher verwendeten Behälter für Ethylenoxid/R 12-Gemische verwendet werden.

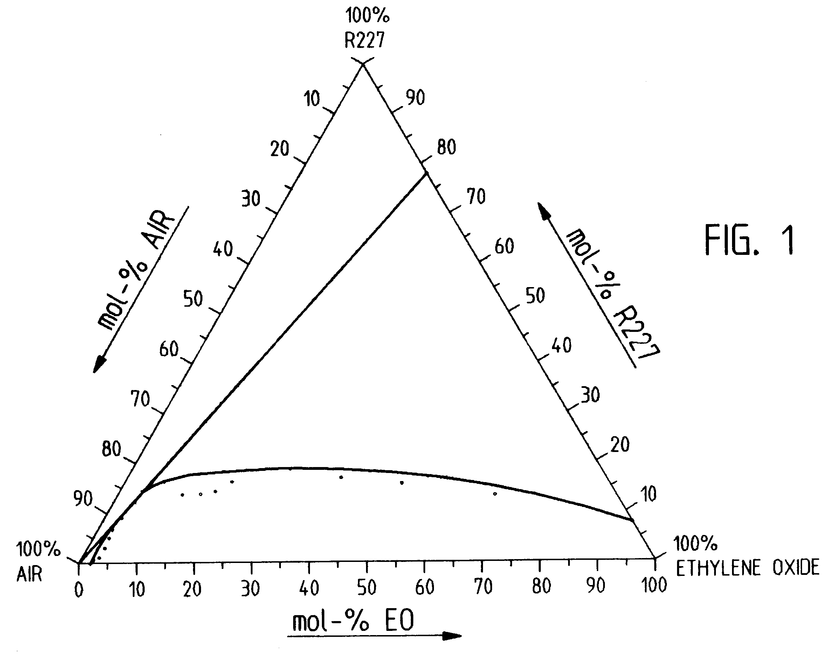

Die Bestimmung der Zündgrenzen des erfindungsgemäßen Sterilisiergasgemisches erfolgte in einer Apparatur nach DIN 51 649 (Teil 1). Zur Bildung verschiedener Konzentrationen des Gasgemisches wurde eine Wösthoff-Gasmischpumpe sowie für die Zuführung der Luft ein Massendurchflußmesser der Fa. Brooks benutzt. Die Temperatur im Zündgefäß betrug 65°C; die Versuche wurden bei Normaldruck durchgeführt. Die Ergebnisse der Zündgrenzenbestimmungen des ternären Systems Alkylenoxid/R 227/Luft wurden im Dreiecksdiagramm (Fig. 1) grafisch dargestellt. In Fig. 1 sind die Einzelwerte und eine Ausgleichskurve eingetragen. Diese Ausgleichskurve stellt den Zündgrenzenverlauf für Alkylenoxid/R 227/Luft-Gemische dar.

In die Fig. 1 ist zusätzlich eine Gerade eingezeichnet, deren Ursprung in der linken unteren Ecke (100 mol% Luft) ist und welche die Ausgleichskurve tangiert. Diese Gerade schneidet in Fig. 1 die Achse, welche den Molgehalt von R 227 angibt. Dieser Schnittpunkt gibt das Verhältnis an, bei welchem gerade keine zündfähigen Gemische aus Alkylenoxid und R 227 mehr im Gemisch mit Luft gebildet werden. Aus den 78 mol% R 227 ergeben sich 22 mol% Alkylenoxid als Differenz zu 100 mol%. Gemische mit weniger als 22 mol% Alkylenoxid bilden keine zündfähigen Gemische mit Luft, wenn das Alkylenoxid mit R 227 als Phlegmatisierungsmittel vermischt ist.

Wie vergleichende Untersuchungen mit 1,2,2,2-Tetrafluorethan (R 134 a) als phlegmatisierende Komponente gezeigt haben, wird die Zündgrenze einer solchen Sterilisiergasmischung bereits ab einem Ethylenoxidgehalt von 14 mol% erreicht. Die Phlegmatisierung von Ethylenoxid mit R 134 a ist somit wesentlich schlechter als die mit R 227.

## Patentansprüche

1. Sterilisiergasgemisch für die Kaltgassterilisation, welches kein zündfähiges Gemisch mit Luft bildet, bestehend aus Alkenoxid und 1,1,1,2,3,3,3-Heptafluorpropan.

2. Sterilisiergasgemisch nach Anspruch 1, bestehend aus 12 bis 22 mol% Ethylenoxid und 78 bis 88 mol% 1,1,1,2,3,3,3-Heptafluorpropan.

3. Sterilisiergasgemisch nach Anspruch 1, bestehend aus 12 bis 22 mol% Propylenoxid und 78 bis 88 mol% 1,1,1,2,3,3,3-Heptafluorpropan.

4. Sterilisiergasgemisch nach einem der Ansprüche 1 bis 3, dadurch gekennzeichnet, daß das Alkenoxid eine Mischung aus Ethylenoxid und Propylenoxid ist.

5. Sterilisiergasgemisch nach einem der Ansprüche 1 bis 4, dadurch gekennzeichnet, daß es Inertgase in Mengen bis zu 2° Volumen% enthält.

6. Sterilisiergasgemisch nach Anspruch 5, dadurch gekennzeichnet, daß das Inertgas Stickstoff, Kohlendioxid oder Edelgas ist.

7. Verwendung eines Sterilisiergasgemisches nach einem der Ansprüche 1 bis 6, zur Sterilisierung von medizinischen Ausrüstungen und Materialien.

8. Verwendung eines Sterilisiergasgemisches nach einem der Ansprüche 1 bis 6, zur Sterilisierung von Fellen, Papiererzeugnissen, Transportbehältern oder zur Sterilisierung von Frachträumen von Flugzeugen, Zügen und Schiffen.

9. Verwendung von 1,1,1,2,3,3,3-Heptafluorpropan als Phlegmatisierungsmittel, zur Herstellung von Sterilisiergasgemischen für die Kaltgassterilisation auf der Basis von Ethylenoxid, welche kein zündfähiges Gemisch mit Luft bilden.

10. Verwendung von 1,1,1,2,3,3,3-Heptafluorpropan als Phlegmatisierungsmittel, zur Herstellung von Sterilisiergasgemischen für die Kaltgassterilisation auf der Basis einer Ethylenoxyd/Propylenoxid Mischung, welche kein zündfähiges Gemisch mit Luft bilden.

## Claims

1. A sterilant gas mixture for cold-gas sterilization, which does not form an ignitable mixture with air consisting of alkene oxide and 1,1,1,2,3,3,3-heptafluoropropane.

2. The sterilant gas mixture as claimed in claim 1, consisting of from 12 to 22 mol % of ethylene oxide and from 78 to 88 mol % of 1,1,1,2,3,3,3-heptafluoropropane.

3. The sterilant gas mixture as claimed in claim 1, consisting of from 12 to 22 mol % of propylene oxide and from 78 to 88 mol % of 1,1,1,2,3,3,3-heptafluoropropane.

4. The sterilant gas mixture as claimed in any of claims 1 to 3 wherein the alkene oxide is a mixture of ethylene oxide and propylene oxide.

5. The sterilant gas mixture as claimed in any of claims 1 to 4, wherein the mixture comprises inert gas in amounts of up to 20 volume %.

6. The sterilant gas mixture as claimed in claim 5, wherein the inert gas in nitrogen, carbon dioxide or noble gas.

7. Use of the sterilant gas as claimed in any of claims 1 to 6 for the sterilization of medical equipments and materials.

8. Use of the sterilant gas mixture as claimed in any of claims 1 to 6, for the sterilization of furs, paper products, transport containers or for the sterilization of freight holds of aeroplanes, trains and ships.

9. Use of 1,1,1,2,3,3,3-heptafluorpropane as inerting agent for the preparation of a sterilant gas mixture for cold-gas sterilization based on ethylene oxide, which does not form an ignitable mixture with air.

10. Use of 1,1,1,2,3,3,3-heptafluorpropane as inerting agent for the preparation of a sterilant gas mixture for cold-gas sterilization based on a mixture ethylene oxide/propylene oxide, which does not form an ignitable mixture with air.

## Revendications

1. Mélange de gaz stérilisant pour la stérilisation à gaz froid, qui ne forme pas de mélange inflammable avec de l'air, constitué d'oxyde d'alcène et de 1,1,1,2,3,3,3-heptafluorpropane.

2. Mélange de gaz stérilisant selon la revendication 1, constitué de 12 à 22% mol d'oxyde d'éthylène et de 78 à 88% mol de 1,1,1,2,3,3,3-heptafluorpropane.

3. Mélange de gaz stérilisant selon la revendication 1, constitué de 12 à 22% mol d'oxyde de propylène et de 78 à 88% mol de 1,1,1,2,3,3,3-heptafluorpropane.

4. Mélange de gaz stérilisant selon l'une quelconque des revendications 1 à 3, caractérisé en ce que l'oxyde d'alcène est un mélange d'oxyde d'éthylène et d'oxyde de propylène.

5. Mélange de gaz stérilisant selon l'une quelconque des revendications 1 à 4, caractérisé en ce qu'il contient des gaz inertes en des quantités jusqu'à 20% en volume.

6. Mélange de gaz stérilisant selon la revendication 5, caractérisé en ce que le gaz inerte est de l'azote, du dioxyde de carbone ou un gaz noble.

7. Utilisation d'un mélange de gaz stérilisant selon l'une quelconque des revendications 1 à 6, pour la stérilisation d'équipements et matériaux médicaux.

8. Utilisation d'un mélange de gaz stérilisant selon l'une quelconque des revendications 1 à 6, pour la stérilisation de fourrures, de produits en papier, de récipients de transport ou pour la stérilisation de soutes de fret d'avions, de trains ou de navires.

9. Utilisation de 1,1,1,2,3,3,3-heptafluorpropane comme agent inertant, pour la fabrication d'un mélange de gaz stérilisant pour la stérilisation à gaz froid à base d'oxyde d'éthylène qui ne forme pas de mélange inflammable avec de l'air.

10. Utilisation de 1,1,1,2,3,3,3-heptafluorpropane comme agent inertant, pour la fabrication d'un mélange de gaz stérilisant pour la stérilisation à gaz froid à base d'un mélange oxyde d'éthylène/oxyde de propylène qui ne forme pas de mélange inflammable avec de l'air.
